# EUROPEAN PATENT APPLICATION

(11) **EP 1 072 890 A2**
(43) Date of publication of application: **31.01.2001**
(21) Application number: 00114157.1
(22) Date of filing: 12.07.2000
(51) Int. Cl.: G01N 33/574

(54) **Method for the detection of prostate cancer**

(30) Priority: 24.07.1999 US 145523 P
(71) Applicant: Bayer Corporation, Pittsburgh, PA 15205 (US)
(72) Inventor: Allard, William Jeffrey, Poughquag, New York 12570 (US)
(74) Representative: Burkert, Frank

(57) **Abstract**

A method for aiding in the detection of prostate cancer in male human patients, e.g., for differentiating prostate cancer from the normal condition and from benign prostate diseases such as prostate hypertrophy (BPH) and prostatitis, by a reflex method in which complexed prostate specific antigen (cPSA) is first measured in a blood sample from a male patient, and if the patient's cPSA blood level is greater than an upper limit of normal (e.g., between about 2 and about 4 ng/mL), total PSA (tPSA) is then measured in the sample and the proportion of the measured tPSA amount that is the measured cPSA amount is determined. The cut-off for the proportion of cPSA to tPSA can be set to provide an indication that the patient is not at risk for prostate cancer (e.g., less than about 82%), or to provide an indication that the patient is at risk for prostate cancer (e.g., greater than about 83%). This c + c/t reflex method has been found to provide an improved method for the detection of prostate cancer, eliminating the need for a significant number of patients to undergo unnecessary prostate biopsy.

## Description

### BACKGROUND OF THE INVENTION

The present invention relates to the detection of prostate cancer in human male patients. More particularly, the invention relates to the differentiation of prostate cancer from the normal condition and from benign prostate diseases, such as benign prostate hypertrophy (BPH) and prostatitis.

The implementation of serum testing for prostate specific antigen (PSA) has led to an increase in prostate cancer (CaP) incidence from 122,000 estimated new cases in 1991 to approximately 200,000 new cases in 1998 (1). While serum PSA testing in conjunction with digital rectal examination and other modalities has high sensitivity for detection of CaP, the specificity is low due to non-cancer specific elevation of PSA in benign prostate diseases. A number of alternative practices have been suggested to improve the specificity of PSA testing including age adjusted reference ranges (2), PSA density (3) or PSA transitional zone density (4), and PSA velocity (5), but none of these approaches has gained widespread acceptance. Another means to improve the specificity of PSA testing results from the work of Stenman (6) and Lilja (7) and others who showed that PSA exists in serum in free form and bound to protease inhibitors, primarily alpha-1-antichymotrypsin. Numerous studies have now established that the proportion of free PSA is reduced in men with prostate cancer (8,9). Results of a recent prospective clinical trial suggested that in men whose total PSA concentrations range between 4 - 10 ng/mL the use of the free/total PSA ratio could reduce unnecessary biopsies by 20% while maintaining 95% sensitivity (10). The implementation of free and total PSA has proven difficult, however, for a number of reasons. First, reflex testing to free PSA is necessary when total PSA is within a specified gray zone, however various recommendations for a gray zone have been suggested over the range of 0 - 20 ng/mL (11,12). Similarly, different kits for free and total PSA have been shown to require different cutoff values for the free/total PSA ratio, and even when using these cutoffs, different levels of specificity have been found (12,13). Free PSA concentrations measured in serum have been found to decrease over time, especially at 4° C, but even at frozen temperatures as well (14).

For these reasons, an alternative to the use of free and total PSA was sought and an accurate and reproducible test for complexed PSA subsequently developed wherein PSA complexed with protease inhibitors is measured to the exclusion of free PSA (15). In a preliminary retrospective trial it was shown that cPSA may provide a stand-alone alternative to the use of a ratio of free and total PSA in the early detection of prostate cancer (16).

However, there is a continuing need for more specific methods for use in the detection of prostate cancer in order to further reduce the number of unnecessary biopsies in the screening of male patients.

U.S. Patent No. 5,501,983 relates to assays for free and complexed PSA and their use in the diagnosis of prostate cancer.

U.S. Patent Nos. 5,698,402 and 5,710,007 relate to reflex methods for use in the diagnosis of prostate cancer.

### SUMMARY OF THE INVENTION

The present invention provides a method for detecting prostate cancer in male human patients. In general, such a method requires the ability to differentiate prostate cancer from the normal condition and from benign prostate diseases, such as benign prostate hypertrophy (BPH) and prostatitis. The present method comprises the steps of (a) measuring the amount of complexed prostate specific antigen (cPSA) in a blood sample obtained from such patient, (b) determining if the patient's cPSA blood level is greater than an upper limit of normal having a value between about 2 and about 4 ng/mL, (C) if said patient's cPSA blood level is greater than said upper limit of normal, measuring the amount of total prostate specific antigen (tPSA) in said blood sample and determining the proportion of the measured tPSA amount that is the measured cPSA amount. The upper limit of normal for the cPSA determination is usually between about 3.0 and about 3.8 ng/mL, preferably between about 3.50 and 3.75 ng/mL; and more preferably is about 3.6 mg/mL. The cut-off for the proportion of cPSA to tPSA can be set to provide an indication that the patient is not at risk for prostate cancer (e.g., the proportion of cPSA is less than a cut-off value set between about 70% and about 82%, and more preferably set at about 75%,), or to provide an indication that the patient is at risk for prostate cancer (e.g., the proportion of cPSA is greater than a cut-off value set between about 83% and about 95 %, and more preferably set at about 90%).

A particularly useful method for measuring cPSA in a blood sample involves treating the blood sample to render uncomplexed, i.e., free, PSA (fPSA) nondetectable by immunoassay, and then determining PSA in the treated blood sample by immunoassay whereby only cPSA is detectable. The immunoassay can be performed in any conventional manner, but more usually is a competitive immunoassay or a two-site immunometric assay. Such method can be accomplished in a variety of ways as described in more detail below. In general, such methods include separation methods in which fPSA is physically removed or retained from the immunoassay test mixture, as well as methods in which an antigenic determinant or determinants in fPSA are modified, such as by chemical interaction, to render fPSA essentially unable to bind to antibody used in the immunoassay method, thereby effectively eliminating fPSA from the assay. Alternatively, cPSA can be measured by a method that is specific for measuring PSA-ACT, for example, without limitation, by a two-site immunometric assay employing an anti-PSA antibody and an anti-ACT antibody, or by a two-site immunometric assay employing an anti-PSA antibody and an antibody that is specific for binding to PSA-ACT complex.

A particularly advantageous two-site immunometric assay method has been devised based on a three antibody reagent system:
(a) a first anti-PSA antibody (monoclonal or polyclonal) which binds to tPSA and which participates in the immunometric assay,
(b) a second anti-PSA antibody (preferably monoclonal) which also binds to tPSA and which also participates in the immunometric assay, but which is selected to have the property that it is substantially incapable of binding to PSA when PSA is bound by a fPSA-specific antibody (this second antibody is referred to herein at times as "MM1"), and
(c) a third anti-PSA antibody which is fPSA-specific, and preferably is monoclonal.

In any of the above described two-site immunometric assays (for cPSA, PSA-ACT or tPSA), one of the first and second antibody reagents can be labeled for detection purposes (and can be referred to as the "labeled" or "detection" antibody) and the other can be immobilized or be capable of being immobilized for purposes of separation from the test mixture (the "capture" antibody).

It has been found that the present method provides an improvement in the detection of prostate cancer in male human patients, increasing the specificity of the determination, thereby reducing the number of unnecessary biopsies in screening for prostate cancer.

### BRIEF DESCRIPTION OF THE DRAWINGS

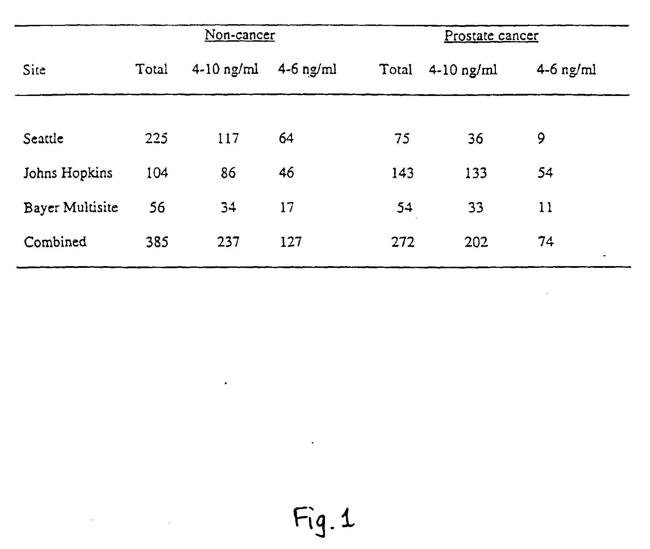

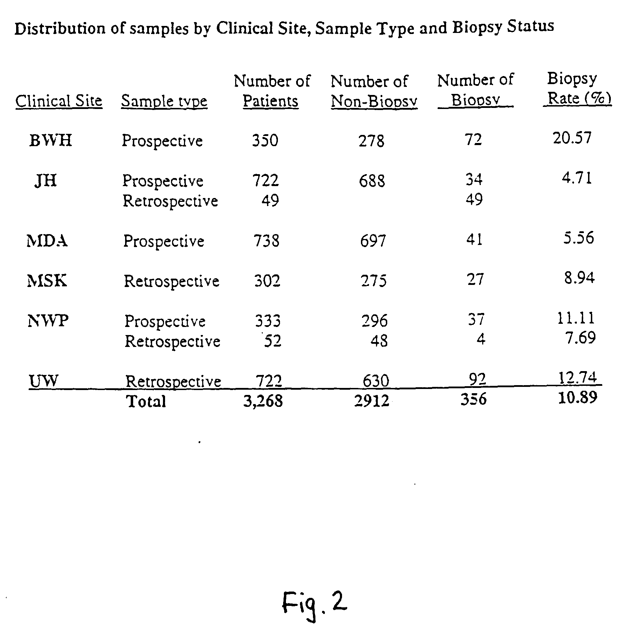

Figs. 1 and 2 are tables showing the sources of the patient samples tested in Studies 1 and 2, respectively, described in the Examples below.

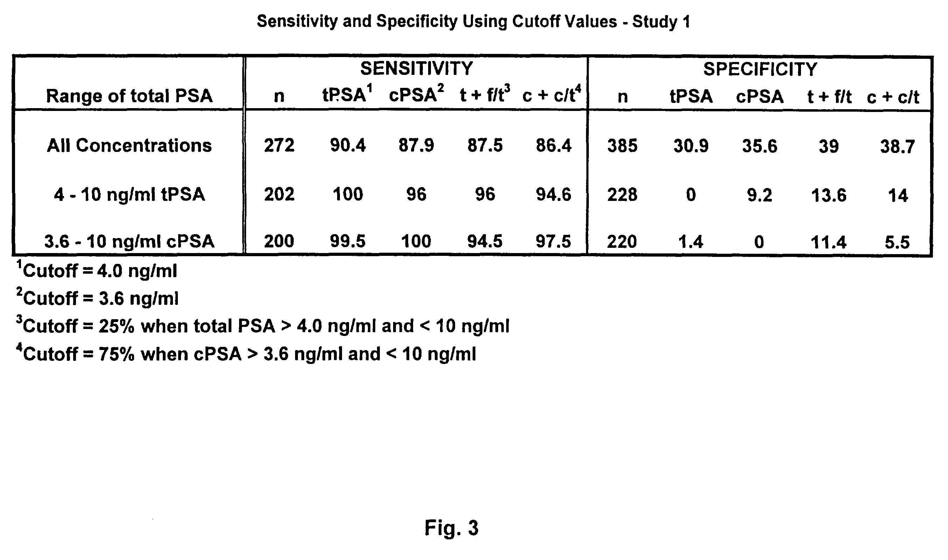

Fig. 3 is a table showing a summary of the sensitivity and specificity for tPSA, cPSA, tPSA together with percent fPSA, and cPSA together with percent cPSA, for patients samples collected in Study 1. tPSA, cPSA and fPSA were measured using the following methods as described in the Examples: tPSA and cPSA by the Bayer method, and percent fPSA was calculated using fPSA and tPSA measured by the Hybritech method.

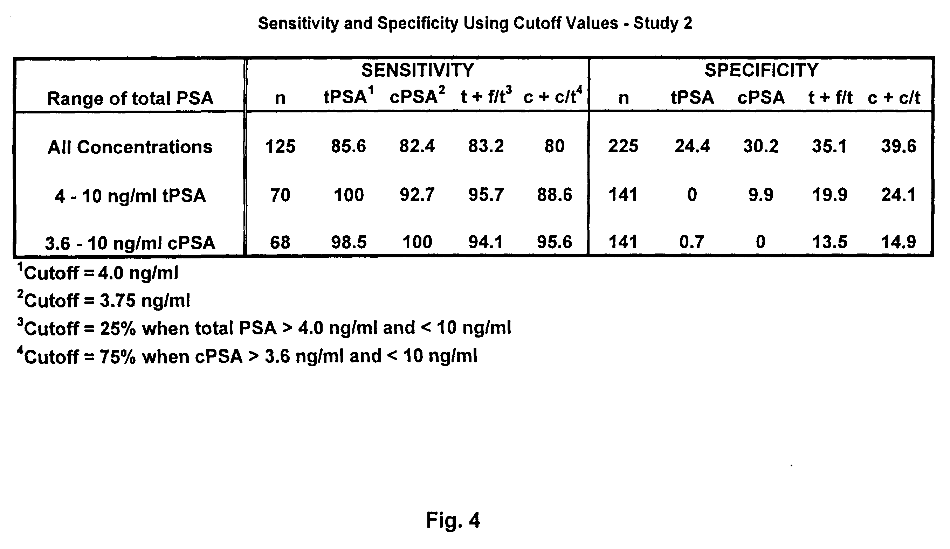

Fig. 4 is a table showing a summary of the sensitivity and specificity for tPSA, cPSA, tPSA together with percent fPSA, and cPSA together with percent cPSA, for patients samples collected in Study 2. tPSA, cPSA and fPSA were measured using the methods as indicated above with respect to the Fig. 3 data.

### DESCRIPTION OF THE PREFERRED EMBODIMENTS

As used herein, the following terms shall have the indicated meanings:
PSA shall mean prostate specific antigen.
tPSA or total PSA shall mean the total amount of immunologically determinable PSA in a blood sample, that is, PSA in complexed or free forms that are capable of responding to measurement by conventional immunoassays. Based on current knowledge, it is understood that blood PSA that is complexed with certain protease inhibitors (including ACT, α₁-antitrypsin, and inter-α trypsin inhibitor) is immunologically determinable, whereas PSA is not determinable when complexed with such other protease inhibitors as α₂-macroglobulin.
fPSA or free PSA shall mean PSA in its free, uncomplexed form.
cPSA or complexed PSA shall mean tPSA that is not fPSA.

Antibody reagent shall mean whole immunoglobulin, e.g., IgG or IgM, or an immunoglobulin fragment comprising an antibody binding site, e.g., Fab, Fab', and F(ab')₂ fragments, or aggregates thereof, or any other molecular form comprising an antibody binding site.

Methods for the determination of tPSA are well-know in the art. It will be understood that preferred methods are those which are known to be substantially accurately in the measurement of tPSA independent of molecular form, i.e., independent of the proportion of fPSA to cPSA in the sample (known as "equimolar" assays).

Methods for the determination of cPSA are also known in the art. Particularly preferred methods are described in U.S. Patent No. 5,840,501 which is hereby incorporated by reference.

The present invention will now be illustrated, but is not intended to be limited by, the following examples.

### EXAMPLES

***Automated Immunoassays for Total and Complexed PSA***. The Bayer Immuno 1™ PSA Assay (Bayer Corporation, Business Group Diagnostics, Tarrytown, New York, USA) is a commercially available sandwich immunoassay which uses a monoclonal antibody for capture and affinity purified goat polyclonal antibodies conjugated to alkaline phosphatase for detection. This assay has been described in detail previously and provides equimolar detection of free and complexed PSA based on the properties of the monoclonal antibody used for capture (17). The Bayer Immuno 1 cPSA assay has been described in detail previously and used the identical antibodies for capture and detection as the total PSA assay, but includes a third unlabeled monoclonal antibody specific for free PSA (15). This third antibody binds to free PSA in the patient sample and renders free PSA immunologically nonreactive. All other conditions were the same as those used in the total PSA assay. The lower limit of detection for these assays is 0.03 ng/mL, and the total CVs range from 2.0-3.4%

***Manual*** ***Immunoassays for Total and Free PSA***. Total PSA was also measured using a double monoclonal antibody sequential radioimmunoassay or a double monoclonal antibody sequential enzyme immunoassay (Tandem-R and Tandem-E assays, Beckman-Coulter), and free PSA was measured using a double monoclonal antibody radioimmunoassay (Tandem-R free PSA assay, Beckman-Coulter) according to the manufacturer's instructions. The lower limit of detection for these assays is 0.3 ng/mL and inter- and intraassay CVs for these assays have been reported to range from 4.8 - 7.7% (12).

***Patient*** ***Samples - Study 1***. Serum samples for this study were collected as part of screening events and archived for further evaluation. Samples were collected prior to prostatic manipulation, and were tested retrospectively at three sites including the University of Washington, Seattle, WA, the Johns Hopkins Hospital, Baltimore, MD, and Memorial Sloan Kettering Cancer Institute, New York, NY, under protocols approved by the Investigational Review Boards at each site. Numbers of specimens from each site are summarized in Figure 1. Samples collected at the University of Washington were selected to provide 25% cancers which approximates the known yield of cancer at that site and results with these patient samples have been presented previously (16). Each patient underwent a sextant transrectal ultrasound-guided prostate needle biopsy. A total of 385 samples were drawn from men with no evidence of malignancy on biopsy (NEM), and 272 samples were from men with cancer. Following centrifugation, all sera were stored at - 70° C and the testing of each sample for total, free and complexed PSA was performed within 2 freeze-thaw cycles.

***Patient*** ***Samples - Study 2***. A total of 3268 serum samples were collected for this study as part of a prospective prostate cancer screening trial where 2143 (66%) samples were collected prospectively and 1125 (34%) were collected from retrospective sample banks. Patients were recruited at The Departments of Urology at Brigham and Women's Hospital, Boston, MA; John Hopkins Hospital, Baltimore, MD; M.D. Anderson Cancer Center, Houston, TX; Memorial Sloan Kettering Cancer Center, New York, NY; Northwest Hospital, Seattle, WA; and the University of Washington, Seattle, WA. Numbers of specimens and biopsy rates are summarized in Figure 2. Patient samples were collected under protocols approved by Institutional Review Boards at each site. Retrospective samples were stored frozen at -70° C, and the testing of all samples for free, total and complexed PSA was done within one freeze thaw cycle. Immunoassays were run at each site using the Bayer Immuno 1 for total and complexed PSA, and manual kits from Hybritech for Tandem total and free PSA.

***Data Analysis***. The Upper Limit used for total PSA was taken from the literature and established as 4.0 ng/mL. For cPSA, the Upper Limit used was 3.6 ng/mL which was calculated to give approximately the same sensitivity for cancer detection as that afforded by total PSA at 4.0 ng/mL. The ratio of free/total PSA has been recommended to reduce unnecessary biopsies by application of the f/t ratio to patients whose total PSA concentrations are in the range of 4.0 - 10.0 ng/mL (10). In this population, a cutoff of 25 % is recommended, such that men with total PSA concentrations between 4 - 10 ng/mL whose f/t PSA ratio (or percent free PSA) is >25%, should not be biopsied. The net effect of this approach is that implementation of the f/t PSA ratio will reduce sensitivity slightly, with a significant improvement in specificity.

To compare the use of complexed PSA and percent complexed PSA to the use of total PSA and percent free PSA, we used an Upper Limit of 3.6 ng/mL for complexed PSA, and an Upper Limit of 75% for the complexed/total PSA ratio. This provided an approximately equivalent level of sensitivity, and therefore allowed a comparison of relative gains in specificity using the 2 different approaches.

***Results**.* Using the Upper Limits as described in Materials and Methods, we compared the sensitivity and specificity using four approaches to the early detection of prostate cancer. Sensitivity and specificity for each approach are summarized for Studies 1 and 2 in Figures 3 and 4, respectively. Total PSA with an Upper Limit of 4.0 ng/mL represents the current Standard of Care for the detection of prostate cancer, used in conjunction with Digital Rectal Examination (DRE). For all samples, total PSA as a single test with an Upper Limit of 4.0 ng/mL, provided sensitivities of 86% and 90%, and specificities of 24% and 31 %. That means that for all patients biopsied in these studies, of the cancers present total PSA detected approximately 88%, but total PSA was falsely elevated in approximately 73 % of men who did not have cancer. In an effort to improve specificity and decrease the numbers of unnecessary biopsies, we compared the sensitivity and specificity of alternative approaches. The first is the use of percent free PSA, which has been recommended as a second test for men with total PSA concentrations in the range of 4 - 10 ng/mL. Sensitivity using this approach decreased 2 - 3% compared to total PSA alone, as expected, to 83% and 88% for the two studies. Specificity, however, improved 8 - 10% for all samples tested. In the recommended total PSA range of 4 - 10 ng/mL for implementation of percent free, however, specificity improved 14 - 20% compared to the use of total PSA as a single test. We also compared the use of complexed PSA as a single test to total PSA alone and found a small decrease in sensitivity of 2 - 3 %, similar to that for percent free PSA, but an increase in specificity of approximately 5 % for all samples. In the total PSA range of 4 -10 ng/mL, cPSA alone improved specificity 9 - 10%.

cPSA as a single test has consistently shown a benefit in specificity compared with total PSA, both in the current studies as well in several previous reports (16, 17, 18, 19). No previous report, however has examined the use of cPSA together with a ratio of complexed to total PSA. When we applied this analysis, we found that the loss of sensitivity is small, 4% - 6%, but the gain in specificity is larger than that seen with any of the other three approaches. The improvement in specificity was 8 - 16% for all samples tested, and 14 - 24% for the truncated total PSA range of 4 - 10 ng/mL (see Figures 3 and 4). This improvement represents a significant clinical improvement in prostate cancer detection because the cPSA assay alone, as previously reported, provides a benefit of approximately 10% in unnecessary biopsies when used as a first test. This would save significant costs to the health care system by the avoidance of further workup of these patients who would be unlikely to benefit from further evaluation and treatment. The further use of a ratio of complexed/total PSA provides additional specificity as reflected in the data presented. Similar benefits were seen when the patient population was subdivided into those men whose cPSA concentrations were in the range of 3.6 - 10 ng/mL, in analogy with the use of the free/total PSA ratio in the total PSA range of 4 - 10 ng/mL.

Several additional advantages are apparent using the complexed PSA and complexed/total PSA ratio as compared with total PSA and the free/total PSA ratio. First, free PSA is not stable in serum and the concentration of free PSA decreases over time due to complexation with alpha-2-macroglobulin (14). Complexed PSA, in contrast, is highly stable in serum (20). In addition, free PSA has been shown to rise anomalously in serum from patients with late stage disease (21). The origin and biochemical nature of the free PSA in late stage prostate cancer sera is not known, but its presence could lead to a misdiagnosis of no cancer, when in fact a patient may have late stage cancer. Lastly, it has been shown that the concentration of PSA in serum varies over time in the same individual due to unknown factors. The majority of this biological variation has been attributed to changes in free PSA (22). Since the majority of PSA in serum is in the complexed form, measurement of this analyte is inherently more accurate than measurement of free PSA which, in healthy men, is present in very low concentrations. Lastly, we have shown that the concentration of free PSA does not change in men with prostate cancer compared with men who do not have cancer (unpublished data). Taken together, these results suggest that cPSA is biochemically more stable, is present in higher concentrations, increases in the serum of men with cancer, and may show less biological variation. The approach of using complexed PSA as a first test followed by percent complexed PSA, provides a significant benefit in prostate cancer detection by decreasing the amount of testing necessary to accurately diagnose disease, and lowering health care costs. It is contemplated that this approach will also make serial measurements of PSA over time more readily interpretable than serial measurements of free PSA.

The present invention has been particularly described and exemplified above. Clearly, many other variations and modifications of the invention can be made without departing from the spirit and scope hereof.

### BIBLIOGRAPHY

1. Cancer facts and figures. 1998. American Cancer Society.
2. Oesterling JE, Jacobsen SJ, Cooner WH. The use of age-specific reference ranges for serum prostate specific antigen in men 60 years old or older. J Urol 1995;153:1160-63.
3. Benson MC, Whang IS, Pantuck A, Ring K, Kaplan SA, Olsson CA, Cooner WH. Prostate specific antigen density: a means of distinguishing prostatic hypertrophy and prostate cancer. J Urol 1992;147:815-16.
4. Djavan B, Zlotta AR, Byttebier G, shariat S. Omar M, Schulman CC, Marberger M. Prostate specific antigen density of the transition zone for early detection of prostate cancer. J Urol 1998;160:411-19.
5. Carter HB, Pearson JD, Metter EJ, Brant LJ, Chan DW, Andres R et al. Longitudinal evaluation of prostate-specific antigen levels in men with and without prostate disease. JAMA 1992;267:2215-20.
6. Stenman U-H, Leinonen J, Alfthan H, Rannikko S, Tuhkanen K, Alfthan O. A complex between prostate-specific antigen and α-1-antichymotrypsin is the major form of prostate-specific antigen in serum of patients with prostatic cancer: assay of the complex improves clinical sensitivity for cancer. Cancer Res 1991;51:222-26.
7. Lilja H, Christensson A, Dahlen U, Matikainen M-T, Nilsson O, Pettersson, Lovgren T. Prostate-specific antigen in serum occurs predominantly in complex with α-1-antichymotrypsin. Clin Chem 1991;37:1618-25.
8. Christensson A, Bjork T, Nilsson O, Dahlen U, Matikainen T, Cockett ATK *et al*. Serum prostate-specific antigen complexed to α1-antichymotrypsin as an indicator of prostate cancer. J Urol 1993;150:100-105.
9. Luderer AA, Chen Y-T, Soriano TF, Kramp WJ, Carlson G, Cuny C *et al*. measurement of the proportion of free to total prostate-specific antigen improves diagnostic performance of prostate-specific antigen in the diagnostic gray zone of total prostate-specific antigen. Urology 1995;46:187-194.
10. Catalona WJ, Partin AW, Slawin KM, Brawer MK, Flanigan RC, Patel A *et al*. Use of the percentage of free prostate-specific antigen to enhance differentiation of prostate cancer from benign prostatic disease. JAMA 1998;279:1542-1547.
11. Vashi AR, Wojno KJ, Henricks W, England BA, Vessella RL, Lange PH *et al*. Determination of the "reflex range" and appropriate cutpoints for percent free prostate specific antigen in 413 men referred for prostatic evaluation using the AxSym system. Urology 1997;49:19-27.
12. Junker R, Brandt B, Zechel C, Assmann G. Comparison of prostate-specific antigen (PSA) measured by four combinations of free PSA and total PSA assays. Clin Chem 1997;43:1588-94.
13. Nixon RG, Meyer GE, Blase AB, Gold MH, Brawer MK. Comparison of 3 investigational assays for the free form of prostate specific antigen. J Urol 1998;160:420-425.
14. Woodrum D, French C, Shamel LB. Stability of free prostate-specific antigen in serum samples under a variety of sample collection and sample storage conditions. Urology 1996;48:33-39.
15. Allard WJ, Zhou Z, Yeung KK. Novel immunoassay for the measurement of complexed prostate-specific antigen in serum. Clin Chem 1998;44:1216-1223.
16. Brawer MK, Meyer JE, Letran JL, Bankson DD, Morris DL, Yeung KK, Allard WJ. Measurement of complexed PSA improves specificity for early detection of prostate cancer. Urology 1998;52:372-378.
17. Allard, WJ, Cheli CD, Neaman IE, Goldblatt J, Morris DL, Smith C, Schwartz MK *et al*. 1999. Complexed PSA and free to total PSA ratio provide equivalent specificity in the detection of prostate cancer but would save biopsies in different patient populations. Clin Chem 45(6):A106.
18. Croal BL, Mitchell IDC, Dickie A, Cohen NP, Duff P, Simpson WG, Ross IS. 1999. Complexed PSA and complexed PSA/prostatic volume ratio in the diagnosis of prostatic carcinoma. Clin Chem 45(6):A108.
19. Allard WJ, Yeung KK, Zhou ZZ. 1998. United States Patent 5,840,501.
20. Allard WJ, Cheli CD, Morris DL, Goldblatt J, Pierre Y, Kish L, Chen Y *et al*. 1999. Multicenter evaluation of the performance and clinical utility in longitudinal monitoring of the Bayer Immuno 1™ complexed PSA assay. Int J Biol Markers14:73-83.
21. Vashi *et al*. F/t PSA ratio is elevated in men with late stage disease.
22. Nixon *et al*. Free PSA is the principle component of variation in biological variation of PSA.

## Claims

1. A method for aiding in the detection of prostate cancer in male human patients, comprising the steps of:
(a) measuring the amount of complexed prostate specific antigen (cPSA) in a blood sample obtained from such patient,
(b) determining if the patient's cPSA blood level is greater than an upper limit of normal having a value between about 2 and about 4 ng/mL, and
(c) if said patient's cPSA blood level is greater than said upper limit of normal, measuring the amount of total prostate specific antigen (tPSA) in said blood sample and determining the proportion of the measured tPSA that is the measured cPSA amount.

2. The method of claim 1 wherein if the proportion of cPSA is less than a cut-off value between about 70% and about 82%, the patient is considered not to be at risk for prostate cancer.

3. The method of claim 2 wherein the upper limit of normal for cPSA is between about 3.0 and about 3.8 ng/mL, more preferably between about 3.50 and about 3.75 ng/mL, and most preferably about 3.6 ng/mL.

4. The method of claim 3 wherein said cut-off is 75%.

5. The method of claim 1 wherein if the proportion of cPSA is greater than a cut-off value between about 83 % and about 95 %, the patient is considered to be at risk for prostate cancer.

6. The method of claim 5 wherein the upper limit of normal for cPSA is between about 3.0 and about 3.8 ng/mL, more preferably between about 3.50 and about 3.75 ng/mL, and most preferably about 3.6 ng/mL.

7. The method of claim 6 wherein said cut-off is 90%.

8. The method of claim 1 wherein cPSA is measured by a method comprising the steps of:
(a) treating tPSA in the blood sample to render free PSA (fPSA) substantially nondetectable by immunoassay, and
(b) determining PSA in the treated blood sample by immunoassay, whereby substantially only cPSA is detectable in said assay.

9. The method of claim 8 wherein step (a) is accomplished by addition to the blood sample of an antibody reagent which modifies antigenic determinants in fPSA sufficiently to render fPSA substantially incapable of binding with antibody used in the immunoassay, and wherein step (b) is performed in the test mixture produced by the addition of the antibody reagent to the blood sample.

10. The method of claim 1 wherein cPSA is measured by a method that is specific for measuring PSA-ACT.
